# EUROPEAN PATENT SPECIFICATION

(11) **EP 1 923 457 B1**
(45) Date of publication and mention of the grant of the patent: **28.06.2017**
(21) Application number: 05775123.2
(22) Date of filing: 18.08.2005
(51) Int. Cl.: A61L 27/56, A61L 27/12, A61L 27/24, C12N 5/00, A61L 27/54, A61L 27/38

(54) **CELL CULTURE SUPPORT EMBEDDABLE IN VIVO**
IN-VIVO-EINBETTBARER ZELLKULTURTRÄGER
SUPPORT DE CULTURE DE CELLULES POUVANT ÊTRE IMPLANTÉ IN VIVO

(43) Date of publication of application: 21.05.2008
(73) Proprietor: KOKEN CO., Ltd., Bunkyo-ku Tokyo 112-0004 (JP)
(72) Inventor: ASO, Yu c/o Laboratory of Koken Co., Ltd., Kita-ku, Tokyo 115-0051 (JP); KONO, Makiko c/o Laboratory of Koken Co., Ltd., Kita-ku, Tokyo 115-0051 (JP)
(74) Representative: Raynor, Stuart Andrew
(86) International application number: PCT/JP2005/015378
(87) International publication number: WO 2007/020713

(56) References cited:
- JP-A- 6 022 744
- JP-A- 6 022 744
- JP-A- 8 038 165
- JP-A- 2003 093 051
- JP-A- 2005 040 060
- JP-A- 2005 130 758
- US-A- 5 024 841
- Hiroshi Itoh ET AL: "A Honeycomb Collagen Carrier for Cell Culture as a Tissue Engineering Scaffold", Artificial Organs, vol. 25, no. 3, 1 March 2001 (2001-03-01), pages 213-217, XP055171189, ISSN: 0160-564X, DOI: 10.1046/j.1525-1594.2001.025003213.x

## Description

### FIELD OF THE INVENTION

The present invention relates to a collagen carrier for cell cultivation which is implantable in vivo. In particular, the present invention relates to a culture carrier, which is usable for regenerative medicine and enables cultivation of cells at high density, characterized by containing a cell to a collagen carrier having fine pores of uniform diameter and orientation, and to a process for preparing said carrier.

### BACKGROUND OF THE INVENTION

As a method for cell cultivation, there are several methods such as to cultivate by adhering a cell to a carrier or to cultivate a cell by floating in a medium. In the case of culturing a cell by adhering to a carrier, the material of the carrier is an important factor to improve the usefulness for high density and efficient culture regenerative medicine.

As a material for a culture carrier, gelatin, cellulose, hydroxy apatite or collagen are known. However from the view point of practical use for regenerative medicine, it is desirable that a compound is easily absorbed in vivo after being embedded. In the case of cellulose, there is a problem that it can not be absorbed in vivo, and from this point of view, collagen attracts attention. Further, for use as a carrier material, the adhering ability of cells and the efficiency must be studied, and collagen has attracted attention from this point of view.

In respect of the shape of the carrier, it is necessary to investigate from a macroscopic view point such as stable preservative ability when the carrier is embedded in vivo, or from a bioactivity view point such as the adhering ability of the cell, bioabsorption·effective bio tissue replacement are important factors. As a bead shaped carrier, for example, a carrier of agarose beads to which collagen is coated is known. However, it has a problem that the binding of the carrier to the embedded part is difficult and abilities of absorbable·effective tissue replacement in vivo are not so good. In addition, as a carrier for adhering cells, micro carrier, hollow fiber, membrane or nonwoven cloth are known, and these subjects are improved from holding ability of carrier. However, they are not sufficient from said bio activity view point.

In the meanwhile, in recent development of regenerative medicine, it has become popular to use auto or allo cells or cells of another person for medical treatment. For example, cultivated skin or cultivated cartilage to which only epithelium cells in vitro cultivated or fibroblast cell in vitro that produce interstitial compound in vivo are introduced are developed and are in practical use.

In these regenerative medicines, after cells are cultivated by suitable carriers, it is clearly understood that the use of a cultivated cell which can be put back in vivo together with the carrier is desirable.

Further, as a carrier having said desired characteristic for regenerative medicine, it is considered that such subject can be accomplished by combining a cell, a carrier for the cell and an activated factor to promote effective cultivation of cell. For example, in a case of bone or cartilage transplantation, it is important that it further contains sufficiently a matrix component (interstitial compound), such as apatite, collagen or mucopolysaccharide and others. Bone or cartilage is composed of apatite, collagen, mucopolysaccharide or others which is produced by a cell, and as a transplantation fragment for these tissues, it is necessary to contain a sufficient amount of matrix component and to have physical characteristics similar to those found in vivo.

Furthermore, because it replaces the patient's own tissue in vivo, matrix components become very important for the purpose to act as an excellent scaffold for cultivation of the patient's own cells. In such a requirement, a spongy carrier is proposed (JP62-502936 publication; WO86/05811, document 1), because a spongy carrier of collagen having an average pore size of approximately 1 µm to 150 µm improves cultivation efficiency. A transplantable transplantation fragment, comprising a carrier with a stable three dimensional structure having inner pores which can pass through nutrient liquid and containing cells in it is proposed (JPH8-511679 publication; WO94/20151, document 2), an implantable material produced from a polymer filament having good shape holding ability (JPH9-182784 publication, document 3), further, a matrix for reproduction of cartilage tissue containing collagen II fiber that composes extracellular matrix having good reproduction ability of cartilage tissue when embedded in vivo (JPH11-503338 publication; WO96/25962, document 4) are proposed. Hiroshi Itoh ET AL: "A Honeycomb Collagen Carrier for Cell Culture as a Tissue Engineering Scaffold",Artificial Organs, vol. 25, no. 3, 1 March 2001 (2001-03-01), pages 213-217,discloses a process for preparing a cell cultivation carrier implantable in vivo having independent pores with opening of 100 µm to 1000 µm on the surface thereof wherein a collagen acidic solution at a concentration of 10mg/ mL, is neutralized' gelled by ammonia gas followed by lyophilization. The resulting structure has a honeycomb structure formed by collagen membranes. Further, a collagen carrier for cell cultivation that can cultivate cells at a high density and a method for production thereof is known. The cell cultivation collagen carrier having a pore size controlled in the 50-2000 µm range, said pore penetrating straight from one surface to another surface and forming collagen fibrils that are arranged straight from one surface to another surface by exposing cell cultivation collagen in which each pores are substantially and independently existing, is prepared by exposing acidic solution to ammonia gas, forming collagen fiber sequenced from one surface to another surface and simultaneously forming a straight water pole from one surface to another surface, then evaporating water in gel by a freeze drying method (Japanese Patent 3170693 registered on March 23, 2001, published on May 28, 2001, document 5). The pore size can be controlled by concentration of ammonia gas and collagen carrier having most suitable pore size and structure to a cell to be cultivated and said pores are controlled by means of the supplying direction of ammonia gas, further the surface area of it can be enhanced. Therefore, a cell cultivation collagen carrier that can cultivate cells by high density may be obtained.

Up to this time, an investigation of a carrier as a scaffold for cells was carried out. However, a report of a practical carrier for regenerative medicine aiming self accumulation of matrix component produced from one's own cell has not been found.

Further, as a factor for activation of cell to said regenerative medicine, factors such as BMP (Bone Morphogeneic Protein), *β* FGF(*β* fibroblast growth factor) are known, and recently, recombinant human factors can be used. Further, these factors can be extracted from animal's bone such as bovine and can be used. However, with any kind of activation factor, it is important for formation of tissue based on said activation factor to maintain said activation factor at a certain concentration at surrounding part.

Recently, big expectation is loaded on regenerative medicine, and development relating to regenerative medicine is becoming an important target, and many firms are joining to the development. However, desirable carriers for regenerative medicine have not yet been developed, and it is a realistic current problem.

The subject of the present invention is to provide a carrier characterizing, after adhering removed cell or tissue thereof to a carrier and cultivating said removed cell or tissue thereof, transforming the carrier to have a figure·structure that may be transplanted in vivo without anaplasia of the cells and so that the matrix produced by the cell is accumulated in sufficient amounts in the patient's own tissue.

For the purpose of solving said subject, the inventors of the present invention basically investigated collagen itself, and considered the development of collagen that can form a cell cultivation carrier, and said collagen can form a carrier having a figure·structure making it possible to accumulate matrix in sufficient amounts in the patient's own tissue in vivo, namely, collagen having a fine pore structure controlled in the thickness direction. Based on said consideration, many experiments were carried out by trial and error, and the inventors of the present invention found out that the neutralization·gellation can be more effectively advanced by controlling the fine pore size and progressing direction, and good effects can be accomplished by transforming soluble collagen to a collagen from which aggregated collagen is removed. For example, the inventors of the present invention found out that by progressing the thickness direction by controlling diffusion, a carrier having a figure·structure characterized in that fine pores having uniform fine pore diameter are uniformly sequenced can be obtained, and in said figure·structure, the matrix can be accumulated in the patient's own tissue in vivo.

### DISCLOSURE OF THE INVENTION

The invention provides (1) a process for preparing a cell cultivation carrier implantable *in vivo* having independent pores with opening of 100 µ m to 1000 µm on the surface thereof wherein collagen acidic solution is preliminary prepared by passing through a filter having a pore size of 10 µm or less and 0.2 µm or more, at a concentration of 5 to 20mg/mL, followed by neutralization·gellation of the collagen acidic solution, wherein basic gas is used at neutralization·gellation process.

The invention further provides (2) a process according to (1), wherein the collagen acidic solution is freeze dried after neutralization· gellation.

The invention further provides (3) a process according to (1), wherein the neutralization is carried out to have directionality at neutralization·gellation process.

The invention further provides (4) a process according to (3), wherein the collagen acidic solution is freeze dried after neutralization· gellation.

The invention further provides (5) a process according to (1), wherein the basic gas is ammonia gas.

The invention further provides (6) a process according to (5), wherein the collagen acidic solution is freeze dried after neutralization· gellation.

The invention further provides (7) a process according to (3), wherein said cell cultivation carrier implantable in vivo has a collagen membrane with pores formed in the neutralization·gellation direction.

The invention further provides (8) a process according to (7), wherein the collagen acidic solution is freeze dried after neutralization· gellation.

The invention further provides (9) a cell cultivation carrier implantable in vivo having independent pores with opening of 100 µm to 1000 µm on the surface thereof, which is obtainable by a process according to any one of (1) to (8).

The invention further provides (10) a cell cultivation carrier according to (9), wherein said cell cultivation carrier implantable *in vivo* is filled with a 2^{nd} component that promotes biochemical tissue in an independent pore.

The invention further provides (11) a cell cultivation carrier according to (10), wherein the 2^{nd} component is hydroxyapatite.

The invention further provides (12) a cell cultivation carrier according to (11), wherein said cell cultivation carrier implantable *in vivo* contains an activation factor for a cell.

### BRIEF ILLUSTRATION OF DRAWINGS

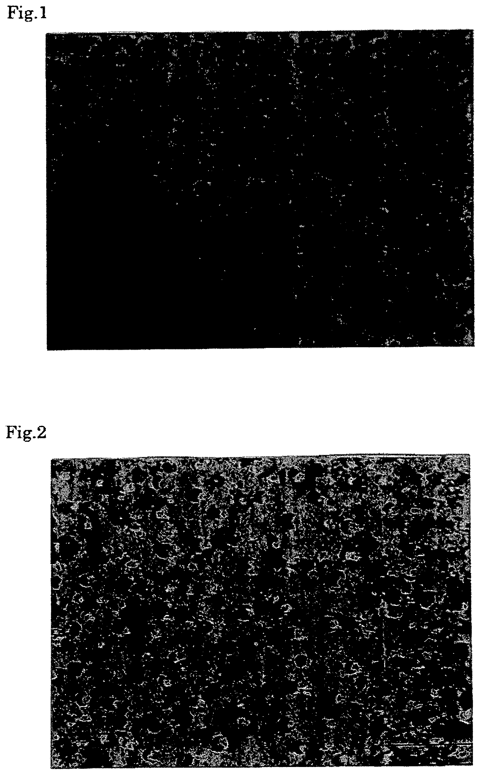

Fig.1 shows a porous spongy carrier having uniform pore size and sequence obtained in Example 1 by following process. That is, ammonia gas is supplied by controlled speed from upper surface to enzyme soluble collagen (atelocollagen) solution obtained by press (max. 4 atom) filtration using membrane filter (product of Millipore Co., Ltd., pore size is 1.0 µm), by controlling neutralization·solidification.
Fig.2 shows a carrier obtained by neutralization·solidification using same process to Example 1except omitting filtration by membrane filter (product of Millipore Co., Ltd., pore size is 1.0 µm) under pressing condition. Unevenness of pore size is large.

### The present invention will be illustrated in more detail

A. The essential factor of the present invention is to use a product that passed through a filter of pore size of 10 µm or less and 0.2 µm or more. This provides collagen, which is characterized in that the molecular weight is approximately 300,000, length is 300nm, diameter is 1.5nm and is rod shaped. Most parts of the molecule form intermolecular crosslinking and result in a large protein of macro molecular weight, so it is necessary to collect a fraction with less intermolecular crosslinking and soluble parts at least in acid. Or, alternatively a method to cut said intermolecular crosslinking, and to extract usable parts must be included.
   A further problem is that even if the protein seems to form a solution, the collagen exists by crosslinking several numbers of molecules. To produce the carrier of the present invention, collagen aggregation of macro molecules should be avoided. The reason why is that when such aggregation of macro molecules is contained, desired characteristics can not be obtained in the neutralization·gellation process.
   Accordingly, passing the material through a filter is necessary to remove such aggregation of macro molecules, and desirable pore size of the filter is 10 µm or less, and 2 µm or more.
   Since the existing state of the collagen molecule depends on the solution condition, it is important to restrict the filtering condition to 5-20mg/mL concentration of collagen acidic solution and to use a filter with pore size of less than 10 µm and 2 µm or more. When the concentration is high, it becomes difficult to pass through the filter even if the collagen is dispersed in the molecular state, and when the concentration is low, although the macromolecules can be removed, the removing effect is too low and causes a problem of productivity. As a pH of acidic condition, 1.5-4.5 is desirable. The kind of filter is not restricted as long as the pore size is 10 µm or less and 2 µm or more.
   The type of collagen to be used is not restricted as long as it can carry out above mentioned treatment. For example, acid soluble, salt soluble or enzyme soluble type I, II or III collagen can be used. However, an enzyme soluble collagen (atelocollagen), which has particularly excellent biocompatibility is desirable. Further, regarding the origin of the collagen, if the above mentioned treatment is possible, both animal originating collagen or recombinant collagen can be used.
B. As the next step, collagen gel is produced by neutralizing the basic solution after said filtering treatment. As a pH of the acidic solution, less than 4.5 in which collagen fibrils can be regenerated is desirable. Filtrated solution can be neutralized as is, or the said solution can be diluted or concentrated so as to change the concentration of collagen. By changing the concentration, it becomes possible to control the physical strength of the finally produced carrier.
C. Neutralization is carried out by exposing the acidic solution to a basic gas When the concentration of collagen is changed use of a basic gas avoids changing the gelling condition. Furthermore, it is desirable to carry out the neutralization to a certain direction, such as from one surface to another surface. By this method, it becomes possible that the formed collagen fiber has directionality, and this is desirable. Specifically, in the case of basic gas, neutralization progresses gradually from the surface exposed to gas to another surface.
   This neutralization diffusion controlling reaction by basic gas is considered to cause organization of the collagen molecule in the gellation process of collagen and brings control of the structure.
   Said neutralization can be observed by confirming that collagen fibers are separated and whitened, or can be observed by carrying out neutralization with mixing an indicator with the acidic solution of collagen.
   As a basic gas, ammonia gas, methylamine or ethylamine can be mentioned. By this neutralization, openings of 50 µm -100 µm can be formed at the surface of the structure, and the openings can be present so that the pores accumulate regularly like a honeycomb structure or can be present so that the pores are arranged at random.
   Further, the pores can penetrate from one opening of one surface to another surface, or can be a blind pore not penetrating from one surface to another surface.
   Above mentioned neutralized collagen gel can be used as is for cultivation or can be used as a spongy product after freeze dried.
D. Carrier concurrently transplantation fragment of this invention is a spongy structural tissue whose starting material is collagen, and can be used by filling with a 2^{nd} component that promotes tissue regeneration in the pore. As the 2^{nd} component, a material that is excellent in cell cytotropic ability such as chitosan, poly lactic acid or poly glycolic acid can be used, in particular, hydroxy apatite is most desirable. As a shape, besides the shape of the pore, any kinds of shape such as granule shape or film shape, which can be filled in the pore can be used, and the granule shape is particularly desirable.
E. Further, it is possible to use the carrier by mixing a cell activation factor. Specifically, it is possible to absorb a bone forming factor by soaking the carrier into solution such as BMP or FGF, or to produce the carrier by mixing a bone forming factor with the collagen material. Furthermore, it is possible to use 2 or more cell activation factors.
   According to the condition of neutralization, sometimes, a big pore is formed in a gel after neutralization, and in said case it becomes hard to cultivate a cell. Therefore, it is desirable for a membrane to be included in the vertical direction to the neutralization direction for the purpose of protecting falling down of a seeding cell. This is because pores are formed to neutralization direction, and the seeding cell is trapped by the membrane, then it becomes possible to be adhered to a wall of pore and multiplied. Specifically, the membrane is separately prepared and it is possible to put it on the neutralized gel, or to put the neutralized gel on it or to put the membrane between formed gels.
F. The carrier concurrently transplantation fragment of this invention can be used after being dried or in the gel state as is. As a method for drying, a freeze dry method is desirable. Cells such as fibroblast, osteoblast, cartilage cell necessary for regenerative medicine or precursor cells of these cells may be seeded to the carrier concurrently transplantation fragment, and cultivated, multiplied by ordinary methods, then transplanted to the appropriate part of a body and used.

### EXAMPLES

Present invention will be illustrated in detail according to Examples. These Examples are intending to make more clear the usefulness of this invention, and are not intended to restrict the scope of this invention. Example 1;

Acidic solution of enzyme soluble collagen (atelocollagen) (concentration of collagen: 11.0mg/mL, pH:3.0) is filtrated by air pressure (max. 4 atoms.) using a membrane filter (product of Millipore, pore size is 1.0 µm). Then the obtained filtrated solution is neutralized and gellated. Specifically, filtrated atelocollagen solution is poured into a tray of 10cm X 10cm size to a height to be 1cm. This tray is contained into a closed container (volume:1L) and ammonia gas flown from a bombe is inserted and filled up. After one hour, the tray is taken out. The atelocollagen solution is gellated and whitened, and water pole phase consisting of water only is formed on the gel toward from upper surface to lower surface and gel is not contained in this phase. Picture showing the surface of obtained carrier that has an opening (SEM picture by electron microscope, magnification is 4) is shown in Fig.1.

This obtained gel is washed by water and dried by freeze dry method, thus a porous sponge having pores of one certain direction.

### Comparative Example

Porous atelocollagen gel is produced by same procedure except carrying out pressing filtration using a membrane filter, and compared with the porous gel produced in Example 1.
In the gel obtained in Example 1, it is confirmed that the pore size locating of upper surface of sponge is uniform, on the contrary, in Comparative Example, unevenness of pore size is large and causes a problem at cell cultivation that needs strictly uniform pore size. Picture showing the surface of obtained carrier that has an opening of Comparative Example (SEM picture by electron microscope, magnification is 4) is shown in Fig.2.

### INDUSTRIAL APPLICABILITY

By using acidic collagen solution which is characterized to be carried out above mentioned filtration, further by combining said collagen solution with specific neutralizing·gelling condition that can control the coagulation of collagen in said collagen solution, a carrier that is possible to cultivate cells suited to regenerative medicine effectively and by high concentration can be provided. Said carrier is usable in the field of regenerative medicine.

## Claims

1. A process for preparing a cell cultivation carrier implantable *in vivo* having independent pores with opening of 100 µm to 1000 µm on the surface thereof wherein a collagen acidic solution is preliminary prepared by passing through a filter having a pore size of 10 µm or less and 0.2 µ m or more, at a concentration of 5 to 20mg/mL, followed by neutralization· gellation of the collagen acidic solution, wherein basic gas is used at neutralization·gellation process.

2. A process according to claim 1, wherein the collagen acidic solution is freeze dried after neutralization·gellation.

3. A process according to claim 1, wherein the neutralization is carried out to have directionality at neutralization·gellation process.

4. A process according to claim 3, wherein the collagen acidic solution is freeze dried after neutralization·gellation.

5. A process according to claim 1, wherein the basic gas is ammonia gas.

6. A process according to claim 5, wherein the collagen acidic solution is freeze dried after neutralization·gellation.

7. A process according to claim 3, wherein said cell cultivation carrier implantable in vivo has a collagen membrane with pores formed in the neutralization·gellation direction.

8. A process according to claim 7 wherein the collagen acidic solution is freeze dried after neutralization·gellation.

9. A cell cultivation carrier implantable in vivo having independent pores with opening of 100 µm to 1000 µm on the surface thereof, which is obtainable by a process according to any one of claims 1 to 8.

10. A cell cultivation carrier according to claim 9, wherein said cell cultivation carrier implantable *in vivo* is filled with a 2^{nd} component that promotes biochemical tissue in an independent pore.

11. A cell cultivation carrier according to claim 10, wherein the 2^{nd} component is hydroxyapatite.

12. A cell cultivation carrier according to claim 11, wherein said cell cultivation carrier implantable *in vivo* contains an activation factor for a cell.

## Patentansprüche

1. Verfahren zur Zubereitung eines Zellkultivierungsträgers, der in vivo implantierbar ist und unabhängige Poren mit einer Öffnung von 100 µm bis 1000 µm an der Oberfläche davon hat, wobei eine saure Kollagenlösung vorher zubereitet wird, indem sie mit einer Konzentration von 5 bis 20 mg/ml durch einen Filter geleitet wird, der eine Porengröße von 10 µm oder weniger und 0,2 µm oder mehr hat, gefolgt von einer Neutralisierungsgelierung der sauren Kollagenlösung, wobei im Neutralisierungsgelierungsverfahren ein basisches Gas verwendet wird.

2. Verfahren nach Anspruch 1, wobei die saure Kollagenlösung nach der Neutralisierungsgelierung gefriergetrocknet wird.

3. Verfahren nach Anspruch 1, wobei die Neutralisierung durchgeführt wird, um im Neutralisierungsgelierungsverfahren eine Ausrichtung zu haben.

4. Verfahren nach Anspruch 3, wobei die saure Kollagenlösung nach der Neutralisierungsgelierung gefriergetrocknet wird.

5. Verfahren nach Anspruch 1, wobei das basische Gas Ammoniakgas ist.

6. Verfahren nach Anspruch 5, wobei die saure Kollagenlösung nach der Neutralisierungsgelierung gefriergetrocknet wird.

7. Verfahren nach Anspruch 3, wobei der *in vivo* implantierbare Zellkultivierungsträger eine Kollagenmembran mit gebildeten Poren in der Neutralisierungsgelierungsrichtung hat.

8. Verfahren nach Anspruch 7, wobei die saure Kollagenlösung nach der Neutralisierungsgelierung gefriergetrocknet wird.

9. Zellkultivierungsträger, der *in vivo* implantierbar ist, mit unabhängigen Poren mit einer Öffnung von 100 µm bis 1000 µm an der Oberfläche davon, der durch ein Verfahren nach einem der Ansprüche 1 bis 8 erlangt werden kann.

10. Zellkultivierungsträger nach Anspruch 9, wobei der *in vivo* implantierbare Zellkultivierungsträger mit einer 2. Komponente gefüllt ist, die biochemisches Gewebe in einer unabhängigen Pore fördert.

11. Zellkultivierungsträger nach Anspruch 10, wobei die 2. Komponente Hydroxyapatit ist.

12. Zellkultivierungsträger nach Anspruch 11, wobei der *in vivo* implantierbare Zellkultivierungsträger einen Aktivierungsfaktor für eine Zelle enthält.

## Revendications

1. Procédé de préparation d' un support de culture cellulaire implantable *in vivo* doté de pores indépendants présentant des ouvertures de 100 µm à 1 000 µm à la surface de celui-ci , dans lequel une solution acide de collagène est préalablement préparée par passage par un filtre dont la taille des pores est de 10 µm ou moins et de 0,2 µm ou plus, à une concentration de 5 à 20 mg/ml, puis par gélification par neutralisation de la solution acide de collagène, dans laquelle un gaz basique est utilisé dans le procédé de gélification par neutralisation.

2. Procédé selon la revendication 1, dans lequel la solution acide de collagène est lyophilisée après la gélification par neutralisation.

3. Procédé selon la revendication 1, dans lequel la neutralisation est effectuée pour obtenir une directionalité dans le procédé de gélification par neutralisation.

4. Procédé selon la revendication 3, dans lequel la solution acide de collagène est lyophilisée après la gélification par neutralisation.

5. Procédé selon la revendication 1, dans lequel le gaz basique est le gaz ammoniac.

6. Procédé selon la revendication 5, dans lequel la solution acide de collagène est lyophilisée après la gélification par neutralisation.

7. Procédé selon la revendication 3, dans lequel ledit support de culture cellulaire implantable *in vivo* présente une membrane de collagène dotée de pores formés dans le sens de la gélification par neutralisation.

8. Procédé selon la revendication 7, dans lequel la solution acide de collagène est lyophilisée après la gélification par neutralisation.

9. Support de culture cellulaire implantable *in vivo* doté de pores indépendants présentant des ouvertures de 100 µm à 1 000 µm à la surface de celui-ci, que l'on peut obtenir par un procédé selon l'une quelconque des revendications 1 à 8.

10. Support de culture cellulaire selon la revendication 9, dans lequel ledit support de culture cellulaire implantable *in vivo* est rempli d'un second composant qui favorise un tissu biochimique dans un pore indépendant.

11. Support de culture cellulaire selon la revendication 10, dans lequel le second composant est l'hydroxyapatite.

12. Support de culture cellulaire selon la revendication 11, dans lequel ledit support de culture cellulaire implantable *in vivo* contient un facteur d'activation pour une cellule.
